(19) Europäisches Patentamt
European Patent Office
Office européen des brevets

(11) **EP 2 432 518 B1**

(12) **EUROPÄISCHE PATENTSCHRIFT**

(45) Veröffentlichungstag und Bekanntmachung des Hinweises auf die Patenterteilung:
**08.01.2014  Patentblatt 2014/02**

(21) Anmeldenummer: **10721324.1**

(22) Anmeldetag: **15.05.2010**

(51) Int Cl.:
***A61M 1/34*** *(2006.01)*

(86) Internationale Anmeldenummer:
**PCT/EP2010/002990**

(87) Internationale Veröffentlichungsnummer:
**WO 2010/133319 (25.11.2010 Gazette 2010/47)**

(54) **VORRICHTUNG UND VERFAHREN ZUR ERKENNUNG EINES SCHLAUCHLEITUNGSSYSTEMS FÜR EINE EXTRAKORPORALE BLUTBEHANDLUNGSVORRICHTUNG**

APPARATUS AND METHOD FOR IDENTIFYING A TUBING SYSTEM FOR AN EXTRACORPOREAL BLOOD TREATMENT DEVICE

DISPOSITIF ET PROCÉDÉ POUR RECONNAÎTRE UN SYSTÈME DE CONDUITE FLEXIBLE POUR UN DISPOSITIF DE TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **19.05.2009   DE 102009021995**

(43) Veröffentlichungstag der Anmeldung:
**28.03.2012   Patentblatt 2012/13**

(73) Patentinhaber: **Fresenius Medical Care Deutschland GmbH**
**61352 Bad Homburg v.d.H. (DE)**

(72) Erfinder:
• **NÜRNBERGER, Thomas**
**97705 Burkardroth (DE)**
• **KLÖFFEL, Peter**
**97720 Nüdlingen (DE)**

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
EP-A1- 0 467 805      EP-A2- 0 238 809
EP-B1- 1 112 099      US-A- 5 644 402

**Beschreibung**

Die Erfindung betrifft eine Vorrichtung zur extrakorporalen Blutbehandlung mit einer Einrichtung zur Erkennung eines in die Blutbehandlungsvorrichtung einzulegenden Schlauchleitungssystems. Darüber hinaus betrifft die Erfindung ein Verfahren zur Erkennung eines in eine extrakorporale Blutbehandlungsvorrichtung einzulegenden Schlauchleitungssystems.

Es sind verschiedene Verfahren zur extrakorporalen Blutbehandlung bekannt. Bei der Hämodialyse (HD) wird das Blut des Patienten in einem extrakorporalen Blutkreislauf gereinigt, der einen Dialysator umfasst. Der Dialysator weist eine Blutkammer und eine Dialysierflüssigkeitskammer auf, die von einer semipermeablen Membran getrennt sind.

Während bei der Hämodialyse (HD) die Dialysierflüssigkeitskammer von Dialysierflüssigkeit durchflossen wird, wobei bestimmte Substanzen auf Grund der Diffusion zwischen der Dialysierflüssigkeit und dem Blut durch die Membran transportiert werden, wird bei der Hämofiltration (HF) die Dialysierflüssigkeitskammer des Dialysators nicht von Dialysierflüssigkeit durchströmt. Bei der Hämofiltration (HF) werden bestimmte Substanzen auf Grund von Konvektion durch die Membran des Filters effektiv entfernt. Eine Kombination aus beiden Verfahren ist die Hämodiafiltration (HDF).

Es ist allgemein bekannt, einen Teil der dem Patienten über die Membran des Dialysators oder Filters entzogenen Flüssigkeit durch eine sterile Substitutionsflüssigkeit zu ersetzen, die entweder stromauf oder stromab des Dialysators dem extrakorporalen Blutkreislauf zugeführt wird. Es sind Vorrichtungen zur extrakorporalen Blutbehandlung bekannt, bei denen die Dialysierflüssigkeit online aus Frischwasser und Konzentraten und die Substitutionsflüssigkeit online aus Dialysierflüssigkeit hergestellt werden.

Für die verschiedenen extrakorporalen Blutbehandlungen werden unterschiedliche zur einmaligen Verwendung bestimmte Schlauchleitungssysteme eingesetzt, die in die Blutbehandlungsvorrichtung eingelegt werden. Für die Behandlung von Erwachsenen oder Kindern beispielsweise sind Schlauchleitungssysteme bekannt, deren Schlauchleitungen sich im Innendurchmesser voneinander unterscheiden.

Die extrakorporalen Blutbehandlungsvorrichtungen verfügen über mehrere Pumpen, mit denen das Blut des Patienten, die Substitutionsflüssigkeit oder Flüssigkeiten zum Spülen in den Schlauchleitungen der Schlauchleitungssysteme gefördert werden.

Zum Fördern von Flüssigkeiten finden bei den extrakorporalen Blutbehandlungsvorrichtungen peristaltische Pumpen Verwendung, bei denen sich mindestens eine Eng- oder Verschlussstelle längs des als Pumpenraum dienenden elastischen Schlauchs bewegt. Bei der gebräuchlichsten Bauart der peristaltischen Schlauchpumpen wird der elastische Schlauch an der Eng- oder Verschlussstelle vollständig verschlossen. Diese Pumpen werden daher auch als okkludierende Schlauchpumpen bezeichnet. Die gebräuchlichste okkludierende Schlauchpumpe ist eine Rollenpumpe, in die ein Abschnitt einer Schlauchleitung des Schlauchleitungssystems eingelegt wird.

Aus der EP 1 112 099 B1 ist eine Vorrichtung zur Überwachung einer Schlauchleitung eines extrakorporalen Blutkreislaufs einer Blutbehandlungsvorrichtung bekannt. Die Überwachungsvorrichtung verfügt über eine Einrichtung, mit der erkannt werden kann, ob in einen Schlauchleitungshalter eine Schlauchleitung eingelegt ist. Die Erkennung der Schlauchleitung beruht darauf, zu überprüfen, ob von einer Lichtquelle ausgesandtes Licht an der Schlauchleitung reflektiert wird. Im Falle einer Lichtreflektion an der Schlauchleitung wird darauf geschlossen, dass eine Schlauchleitung eingelegt ist. Die bekannte Überwachungsvorrichtung erlaubt aber nicht die Erkennung, ob ein bestimmter Schlauchleitungstyp in den Halter eingelegt ist.

Der Erfindung liegt die Aufgabe zu Grunde, eine Vorrichtung zur extrakorporalen Blutbehandlung bereitzustellen, die eine Erkennung des in die extrakorporale Blutbehandlungsvorrichtung einzulegenden Schlauchleitungssystems erlaubt.

Darüber hinaus ist eine Aufgabe der Erfindung ein Verfahren anzugeben, mit dem sich das in die Blutbehandlungsvorrichtung einzulegende Schlauchleitungssystem erkennen lässt.

Die Lösung dieser Aufgaben erfolgt erfindungsgemäß mit den Merkmalen der Patentansprüche 1 und 7. Vorteilhafte Ausführungsformen der Erfindung sind Gegenstand der Unteransprüche.

Die Erkennung des in die extrakorporale Blutbehandlungsvorrichtung einzulegenden Schlauchleitungssystems bietet verschiedene Möglichkeiten zur Vereinfachung der extrakorporale Blutbehandlungsvorrichtung und zur Erhöhung der Sicherheit der extrakorporalen Blutbehandlung. Nach der Erkennung des Schlauchleitungssystems ist es möglich, einen Eingriff in die Maschinensteuerung vorzunehmen. Beispielsweise ist es möglich, nach dem Einlegen eines bestimmten Schlauchleitungssystems nur eine bestimmte Blutbehandlung, beispielsweise nur eine Hämodialyse (HD), nicht aber eine Hämofiltration (HF) oder Hämodiafiltration (HDF) mit der Blutbehandlungsvorrichtung zuzulassen. Es ist aber auch möglich, nach der Erkennung des Schlauchleitungssystems bestimmte Vorgaben für die Blutbehandlung zu machen. Auch kann nach der Erkennung des Schlauchleitungssystems überprüft werden, ob auch das richtige Schlauchleitungssystem für die vorgegebene Blutbehandlung eingelegt ist, beispielsweise kann zwischen einem Schlauchleitungssystem für die Behandlung eines Erwachsenen oder eines Kindes unterschieden werden.

Die Erkennung des Schlauchleitungssystems beruht bei der erfindungsgemäßen Vorrichtung und dem erfindungsgemäßen Verfahren auf der Grundlage der Abhängigkeit der Flussrate, mit der die Flüssigkeit von mindestens einer Pumpe der extrakorporalen Blutbehandlungsvorrichtung in einem Schlauchleitungsabschnitt des Schlauchleitungssystems gefördert wird, von der Drehzahl, mit der die mindestens eine Pumpe betrieben wird, und dem Innendurchmesser der Schlauchleitung des Schlauchleitungsabschnitts. Da die Abhängigkeit der Flussrate von der Pumpendrehzahl und dem Schlauchleitungsquerschnitt bekannt ist, kann darauf geschlossen werden, ob ein bestimmtes Schlauchleitungssystem in die extrakorporale Blutbehandlungseinrichtung eingelegt ist. Der Querschnitt der Schlauchleitung eines Schlauchleitungsabschnitts des Schlauchleitungssystems wird also als Indikator zur Erkennung des Schlauchleitungssystems benutzt.

Mit der erfindungsgemäßen Vorrichtung und dem erfindungsgemäßen Verfahren ist es möglich, zwischen der Schlauchleitung zur Behandlung eines Erwachsenen oder eines Kindes zu unterscheiden, da beide Schlauchleitungssysteme unterschiedliche Schlauchleitungsquerschnitte haben. Es ist aber auch möglich, nur ein Schlauchleitungsabschnitt eines Schlauchleitungssystem mit einem bestimmten Querschnitt zu versehen, der sich von dem Querschnitt eines anderen Schlauchleitungssystem unterscheidet. Dadurch können beide Schlauchleitungssysteme mit der erfindungsgemäßen Vorrichtung oder dem erfindungsgemäßen Verfahren voneinander unterschieden werden. Dabei ist es möglich, einen Schlauchleitungsabschnitt, der nicht für die eigentliche Blutbehandlung, sondern beispielsweise nur zum Spülen verwendet wird, mit einem anderen Innendurchmesser, insbesondere einem verringerten Innendurchmesser, zu versehen. Zur Kennzeichnung des Schlauchleitungssystems durch einen veränderten Querschnitt bietet sich aber auch der Schlauchleitungsabschnitt an, über den Substitutionsflüssigkeit, nicht aber Blut des Patienten zugeführt wird.

Bei den bekannten extrakorporalen Blutbehandlungsvorrichtungen sind während des Spülvorgangs im Allgemeinen zwei Pumpen zum Fördern von Flüssigkeit im Einsatz, die hintereinander angeordnet sind. Die erste Pumpe fördert Flüssigkeit in einem ersten Schlauchleitungsabschnitt und die zweite Pumpe fördert Flüssigkeit in einem zweiten Schlauchleitungsabschnitt des Schlauchleitungssystems. Bei den Pumpen handelt es sich im Allgemeinen um okkludierende Schlauchpumpen, in die der erste und zweite Schlauchleitungsabschnitt eingelegt sind. Beide Schlauchleitungsabschnitte können den gleichen Innendurchmesser aufweisen. Zur Kennzeichnung eines bestimmten Schlauchleitungssystems kann einer der beiden Schlauchleitungsabschnitte aber auch einen größeren oder kleineren Querschnitt aufweisen.

Eine bevorzugte Ausführungsform der Erfindung geht davon aus, dass unter der Voraussetzung, dass die erste und zweite Pumpe Flüssigkeit mit der gleichen Flussrate (Förderrate) fördert, für den Fall gleicher Querschnitte beider Schlauchleitungsabschnitte die Pumpen gleiche Drehzahlen haben und für den Fall unterschiedlicher Querschnitte die Pumpen unterschiedliche Drehzahlen haben. Bei der bevorzugten Ausführungsform wird der Druck in der Schlauchleitung zwischen der ersten und zweiten Pumpe gemessen. Dabei werden die Pumpendrehzahlen so eingestellt, dass der Druck während des Betriebs der Pumpen zumindest in einem vorgegebenen Zeitintervall konstant bleibt. Auf Grund des Verhältnisses der Drehzahlen zwischen der ersten und zweiten Pumpe wird auf gleiche oder unterschiedliche Innendurchmesser der Schlauchleitungen geschlossen. Aus dem Verhältnis der Drehzahlen kann auch der Innendurchmesser der Schlauchleitung eines der beiden Schlauchleitungsabschnitte berechnet werden, wenn der Innendurchmesser der Schlauchleitung des jeweils anderen Schlauchleitungsabschnitts bekannt ist.

Wenn in diesem Zusammenhang von einem konstanten Druck die Rede ist, sind aber in der Praxis Druckpulse zu berücksichtigen, die das im Wesentlichen konstante Drucksignal überlagern. In der Praxis wird daher ein oszillierendes Drucksignal gemessen. Diese Druckpulse sind darauf zurückzuführen, dass die Blutpumpe im Allgemeinen eine Rollenpumpe ist, deren Rollen die Schlauchleitung okkludieren. Die Druckpulse entstehen beim Anheben bzw. Absenken der Rollen auf der Schlauchleitung infolge der Okklusion der Schlauchleitung.

In der Praxis dürfen die Druckpulse für die Auswertung keine Berücksichtigung finden. Hierzu ist es beispielsweise möglich, in einem vorgegebenen Zeitintervall einen mittleren Druck zu berechnen, der konstant sein soll. Die Berechnung des Mittelwerts kann mit der Auswerteinheit erfolgen. Anstelle einer Mittelwertbildung können aber auch obere und/oder untere Grenzwerte vorgegeben werden, wobei ein konstanter Druck angenommen wird, wenn sich das Drucksignal innerhalb des vorgegebenen Grenzwertfensters bewegt. Es ist aber beispielsweise auch möglich, das gemessene Drucksignal von den Druckpulsen zu befreien. Dies ist beispielsweise mit einer Messwertfilterung, insbesondere mit einer Tiefpassfilterung der Messwerte möglich, da die Druckimpulse periodisch auftreten.

Eine alternative Ausführungsform der Erfindung sieht die Erkennung des Schlauchleitungssystems an dem Querschnitt der Schlauchleitung dadurch vor, dass die Flussrate der Flüssigkeit gemessen wird, die in einem Schlauchleitungsabschnitt des Schlauchleitungssystems mit einer Pumpe der extrakorporalen Blutbehandlungsvorrichtung gefördert wird. Die Pumpe wird mit einer Drehzahl betrieben, bei der sich unter der Annahme, dass die Schlauchleitung einen bestimmten Innendurchmesser hat, sich eine bestimmte Flussrate ergibt. Wenn die angenommene Flussrate mit der gemessenen Flussrate identisch ist, wird darauf geschlossen, dass der Innendurchmesser der verwendeten Schlauchleitung mit dem Innendurchmesser der angenommenen Schlauchleitung gleich ist. Damit kann davon ausgegangen werden, dass die richtige Schlauchleitung in die Blutbehandlungsvorrichtung eingelegt ist.

Da in der Praxis davon ausgegangen werden kann, dass sich die Flussrate nicht exakt berechnen bzw. messen lässt, werden bestimmte Abweichungen toleriert. Daher wird vorzugsweise der Betrag der Differenz zwischen den Drehzahlen oder Flussraten oder deren Quotienten mit einem Referenzwert verglichen.

Die erfindungsgemäße Vorrichtung hat den Vorteil, dass die Erkennung des Schlauchleitungssystems mit den Komponenten möglich ist, die im Allgemeinen in den bekannten Blutbehandlungsvorrichtungen ohnehin vorhanden sind. Beispielsweise sind Pumpen zum Fördern von Blut oder Substitutionsflüssigkeit ohnehin vorhanden. Auch ist eine Einheit zum Messen des Drucks in der Schlauchleitung bei den bekannten Blutbehandlungen vorhanden. Die bekannten Blutbehandlungsvorrichtungen verfügen im Allgemeinen auch über eine Einheit, mit der die Flussrate der Flüssigkeit bestimmt werden kann. Zur Messung der Flussrate kann beispielsweise eine Bilanziereinheit verwendet werden, die in Blutbehandlungsvorrichtungen eingesetzt wird.

Im Folgenden werden Ausführungsbeispiele der Erfindung unter Bezugnahme auf die Zeichnungen näher erläutert.

Es zeigen:

Fig. 1    die wesentlichen Komponenten einer Vorrichtung zur extrakorporalen Blutbehandlung mit einer Einrichtung zur Erkennung des Schlauchleitungssystems, wobei mit der extrakorporalen Blutbehandlungsvorrichtung eine extrakorporale Blutbehandlung durchgeführt wird,

Fig. 2    die extrakorporale Blutbehandlungsvorrichtung von Fig. 1, wobei die Blutbehandlungsvorrichtung gespült wird,

Fig. 3    ein Ersatzschaltbild von zwei Schlauchleitungsabschnitten des Schlauchleitungssystems und zwei Pumpen, und

Fig. 4    die Flussraten der Pumpen von Fig. 3 als Funktion der Pumpendrehzahl.

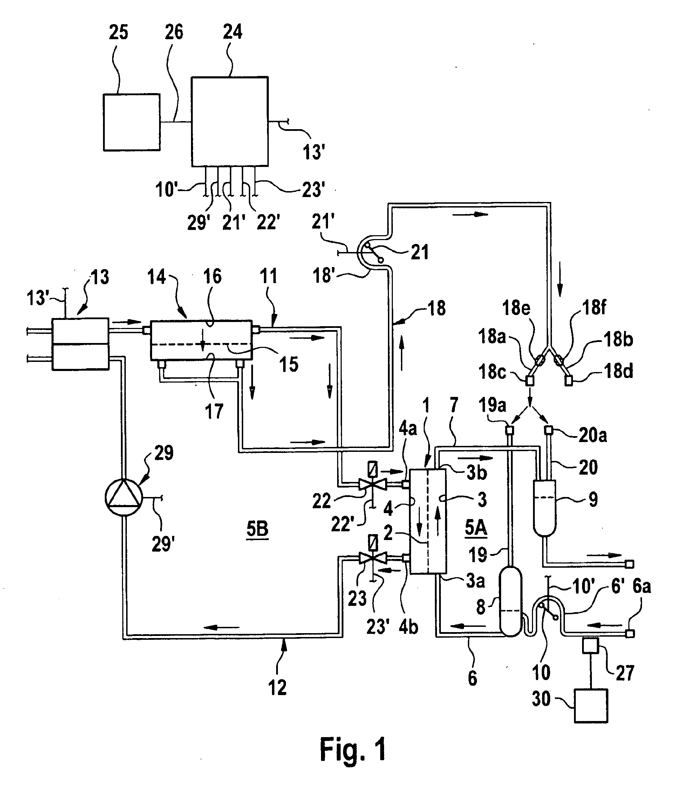

Fig. 1 zeigt eine vereinfachte schematische Darstellung der wesentlichen Komponenten einer extrakorporalen Blutbehandlungsvorrichtung, insbesondere einer Hämo(dia)filtrationsvorrichtung, die über eine Einrichtung zur Erkennung des in die Blutbehandlungsvorrichtung einzulegenden bzw. eingelegten Schlauchleitungssystems verfügt.

Die Hämo(dia)filtrationsvorrichtung weist einen Dialysator 1 auf, der durch eine semipermeable Membran 2 in eine von Blut durchflossene erste Kammer 3 und eine von Dialysierflüssigkeit durchflossene zweite Kammer 4 getrennt ist. Die erste Kammer 3 ist in einen extrakorporalen Blutkreislauf 5A geschaltet, während die zweite Kammer 4 in das Flüssigkeitssystem 5B der Hämo(dia)filtrationsvorrichtung geschaltet ist. Wenn nachfolgend von einem Dialysator die Rede ist, wird darunter auch ein Filter verstanden.

Der extrakorporale Blutkreislauf 5A umfasst eine arterielle Blutleitung 6, die zu dem Einlass 3a der Blutkammer 3 führt, und eine venöse Blutleitung 7, die von dem Auslass 3b der Blutkammer 3 des Dialysators 1 abgeht. Zur Eliminierung von Luftblasen sind bei dem vorliegenden Ausführungsbeispiel in die arterielle Blutleitung 6 eine arterielle Tropfkammer 8 und in die venöse Blutleitung 7 eine venöse Tropfkammer 9 geschaltet. Bei der arteriellen und venösen Blutleitung 6, 7 handelt es sich um zwei separate Schlauchleitungsabschnitte 6, 7 eines zur einmaligen Verwendung bestimmten Schlauchleitungssystems. Beide Schlauchleitungsabschnitte 6, 7 sind bei dem vorliegenden Ausführungsbeispiel über nicht dargestellte Konnektoren an dem Ein- und Auslass 3a, 3b der Dialysierflüssigkeitskammer 3 angeschlossen. Die Schlauchleitungen der beiden Schlauchleitungsabschnitte 6, 7 haben den gleichen Innendurchmesser $d_B$. Ein Leitungsabschnitt 6' des arteriellen Schlauchleitungsabschnitts 6 ist in eine okkludierende Pumpe 10, insbesondere Rollenpumpe eingelegt, die das Blut des Patienten im extrakorporalen Blutkreislauf 5A fördert.

Das Flüssigkeitssystem 5B umfasst eine Dialysierflüssigkeitszuführleitung 11, die zu dem Einlass 4a der Dialysierflüssigkeitskammer 4 führt, und eine Dialysierflüssigkeitsabführleitung 12, die von dem Auslass 4b der Dialysierflüssigkeitskammer 4 des Dialysators 1 abgeht. Über die Dialysierflüssigkeitszuführleitung 11 strömt frische Dialysierflüssigkeit aus einer nicht dargestellten Dialysierflüssigkeitsquelle in die Dialysierflüssigkeitskammer, während verbrauchte Dialysierflüssigkeit aus der Dialysierflüssigkeitskammer über die Dialysierflüssigkeitsabführleitung 12 zu einem nicht dargestellten Abfluss abgeführt wird. Die Dialysierflüssigkeit wird mit einer Dialysierflüssigkeitspumpe 29 gefördert, die in der Dialysierflüssigkeitsabführleitung 12 angeordnet ist. Zur Bilanzierung von frischer zu verbrauchter Dialysierflüssigkeit dient eine Bilanziereinheit 13, die einerseits in die Dialysierflüssigkeitszuführleitung 11 und andererseits in die Dialysierflüssigkeitsabführleitung 12 geschaltet ist, so dass die Bilanzkammern der Bilanziereinheit von zu- und abfließender Dialysierflüssigkeit durchflossen werden.

Während der Blutbehandlung kann eine sterile Substitutionsflüssigkeit dem extrakorporalen Blutkreislauf 5A zugeführt werden. Die sterile Substitutionsflüssigkeit wird aus der Dialysierflüssigkeit des Dialysierflüssigkeitssystems 5B gewonnen. Zur Gewinnung der sterilen Substitutionsflüssigkeit dient ein Sterilfilter 14, der durch eine Membran 15 in eine erste Kammer 16 und eine zweite Kammer 17 unterteilt ist. Die erste Kammer 16 ist in die Dialysierflüssigkeitszuführleitung 11 geschaltet, und von der zweiten Kammer 17 geht eine Substitutionsflüssigkeitsleitung 18 ab, die zu dem extrakorporalen Blutkreislauf 5A führt.

Die Substitutionsflüssigkeitsleitung 18 weist an den Enden zwei Leitungsabschnitte 18a, 18b auf, an denen jeweils ein Konnektor 18c, 18d angeschlossen ist. Mit den beiden Konnektoren 18c, 18d kann die Substitutionsflüssigkeitsleitung 18 an eine zu der arteriellen Tropfkammer 8 führende Anschlussleitung 19 bzw. eine zu der venösen Tropfkammer 9 führende Anschlussleitung 20 angeschlossen werden. Die Anschlussleitungen 19, 20 verfügen daher über entsprechende Anschlussstücke 19a, 20a. Auf den Leitungsabschnitten 18a, 18b sitzen Schlauchklemmen 18e, 18f, mit denen wahlweise eine Flüssigkeitsverbindung zu der arteriellen und/oder venösen Tropfkammer 8, 9 hergestellt werden kann.

Bei der Substitutionsflüssigkeitsleitung 18 handelt es sich wieder um einen separaten Schlauchleitungsabschnitt des Schlauchsystems. Ein Leitungsabschnitt 18' des Schlauchleitungsabschnitts 18 ist in eine Substituatpumpe 21, insbesondere Rollenpumpe eingelegt, mit der die Substitutionsflüssigkeit gefördert wird. Der Schlauchleitungsabschnitt 18 zum Zuführen der Substitutionsflüssigkeit weist einen Innendurchmesser $d_A$ auf, der gleich dem Innendurchmesser $d_B$ der Schlauchleitungsabschnitte der arteriellen und venösen Blutleitung 6, 7 sein kann oder von dem Innendurchmesser $d_B$ der beiden Schlauchleitungsabschnitte 6, 7 verschieden sein kann.

Zum Abklemmen des Dialysators 1 ist in der Dialysierflüssigkeitszuführleitung 11 ein Absperrorgan 22 und in der Dialysierflüssigkeitsabführleitung 12 ein Absperrorgan 23 vorgesehen. Die Blutbehandlungsvorrichtung kann noch über weitere Komponenten, beispielsweise weitere Sterilfilter, Absperrorgane, Konnektoren oder dergleichen verfügen, die aber der besseren Übersichtlichkeit halber nicht dargestellt sind.

Die Steuerung der Blutbehandlungsvorrichtung erfolgt mit einer zentralen Steuereinheit 24, die über Steuerleitungen 10', 29', 21', 22', 23', mit der arteriellen Blutpumpe 10, der Dialysierflüssigkeitspumpe 29, der Substituatpumpe 21 und den Absperrorganen 22 und 23 verbunden ist. Mit der zentralen Steuereinheit 24 können die einzelnen Pumpen ein- und ausgeschaltet sowie deren Drehzahl eingestellt werden.

Die extrakorporale Blutbehandlungsvorrichtung verfügt über eine Einrichtung zum Erkennen des in der Blutbehandlungsvorrichtung eingelegten bzw. einzulegenden Schlauchleitungssystems, das bei dem vorliegenden Ausführungsbeispiel die Schlauchleitungsabschnitte 6, 7, 18 umfasst. Die Einrichtung zur Erkennung des Schlauchleitungssystems weist eine Auswerteinheit 25 auf, die über eine Datenleitung 26 mit der zentralen Steuereinheit 24 der Blutbehandlungsvorrichtung verbunden ist. Die Auswerteinheit 25 kann aber auch Bestandteil der zentralen Steuereinheit 24 sein, da die für die Realisierung der Auswerteinheit notwendigen Komponenten, beispielsweise ein Mikroprozessor oder dergleichen, bereits in der zentralen Steuereinheit vorhanden sind. Neben der Auswerteinheit 25 weist die Einrichtung zur Erkennung des Schlauchleitungssystems eine Druckmesseinheit 27, 30 auf, die mit einem Drucksensor 27 den Druck stromauf der Blutpumpe 10 in der arteriellen Blutleitung 6 misst.

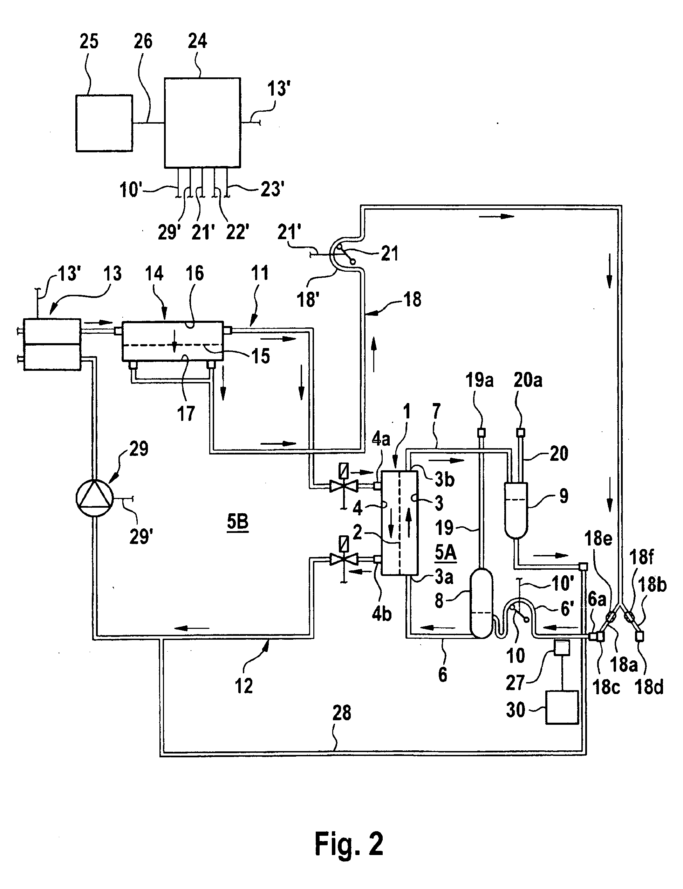

Das Schlauchleitungssystem 6, 7, 18 wird während einer Spülphase erkannt, die der Blutbehandlung vorausgeht. Fig. 2 zeigt die Blutbehandlungsvorrichtung von Fig. 1 während der Spülphase. Zum Spülen der Blutbehandlungsvorrichtung wird die Substitutionsflüssigkeitsleitung 18 nicht mit den zu den Tropfkammern 8 bzw. 9 führenden Anschlussleitungen 19 bzw. 20 verbunden, sondern an das Ende der arteriellen Blutleitung 6 angeschlossen. Hierzu wird der Konnektor 18c mit einem passenden Konnektor 6a verbunden, der am Ende der Blutleitung 6 vorgesehen ist. Weiterhin wird die Schlauchklemme 18e geöffnet und die Schlauchklemme 18f geschlossen. Darüber hinaus werden die Absperrorgane 22 und 23 am Ein- und Auslass 4a, 4b der Dialysierflüssigkeitskammer 4 des Dialysators 1 geschlossen. Folglich kann Spülflüssigkeit über die Dialysierflüssigkeitszuführleitung 11 in den Sterilfilter 14 und über die Substitutionsflüssigkeitsleitung 18 und arterielle Blutleitung 6 in die Blutkammer 3 des Dialystors 1 strömen. Aus der Blutkammer 3 fließt die Spülflüssigkeit in die venöse Blutleitung 7, die in der Spülphase über einen nur andeutungsweise dargestellten Leitungspfad 28 an die Dialysierflüssigkeitsabführleitung 12 angeschlossen ist. Bei dem vorliegenden Ausführungsbeispiel wird angenommen, dass sich der Innendurchmesser $d_A$ des Schlauchleitungsabschnitts der Substitutionsflüssigkeitsleitung 17 von dem Innendurchmesser $d_B$ der beiden Schlauchleitungsabschnitte der arteriellen und venösen Blutleitung 6, 7 unterscheidet. Der Innendurchmesser $d_B$ ist hier größer als der Innendurchmesser $d_A$. Beispielsweise ist der Innendurchmesser $d_B$ gleich 8 mm und der Innendurchmesser $d_A$ 4 mm. Das Schlauchleitungssystem 6, 7, 18 ist also durch Schlauchleitungsabschnitte 6, 7 bzw. 18 unterschiedlichen Innendurchmessers gekennzeichnet. Es ist grundsätzlich auch möglich, dass nur die in die Blutpumpe 10 bzw. Substituatpumpe 21 eingelegten Leitungsabschnitte 6' bzw. 18' der Schlauchleitungsabschnitte 6 bzw. 18 unterschiedliche Innendurchmesser aufweisen, die nicht in den Pumpen liegenden Leitungsabschnitte sich aber im Innendruchmesser nicht voneinander unterscheiden.

Während der Spülphase sind zwei Schlauchleitungsabschnitte 6, 18 unterschiedlichen Innendurchmessers $d_A$, $d_B$ in Reihe geschaltet, wobei in dem Leitungsabschnitt 18 die Spülflüssigkeit mit der Substituatpumpe 21 und in dem Leitungsabschnitt 6 die Flüssigkeit mit der Blutpumpe 10 gefördert wird.

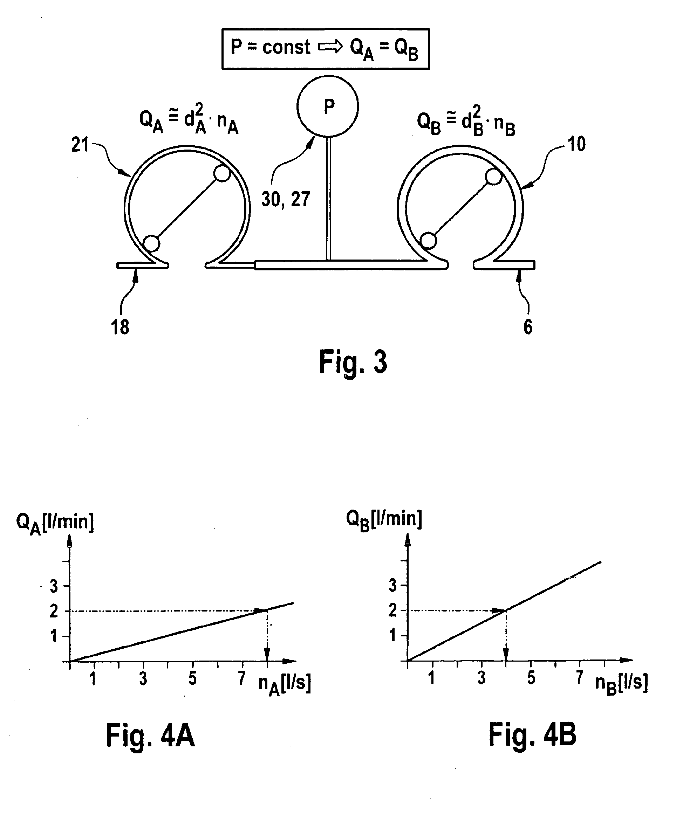

Fig. 3 zeigt ein Ersatzschaltbild mit den beiden Schlauchleitungsabschnitten 18, 6 und den beiden Pumpen 21, 10 sowie der Druckmesseinheit 27, 30 mit dem Drucksensor 27 zum Messen des Systemdrucks P in dem Schlauchleitungsabschnitt 6. Die Erkennung des Schlauchleitungssystems erfolgt durch Konstanthalten des resultierenden Systemdrucks P und ggf. einer Drehzahlnachregelung einer der beiden Pumpen.

Die Förderrate $Q_{A,B}$ der beiden okkludierenden Schlauchpumpen 21, 10, insbesondere Rollenpumpen, hängt vom inneren Schlauchquerschnitt und von der Wandergeschwindigkeit der Eng- bzw. Verschlussstelle der Schlauchpumpen ab. Für die okkludierende Rollenpumpe gilt:

$$Q = V_i \cdot i \cdot n.$$

wobei $V_i$ das zwischen zwei Rollen der Rollenpumpe eingeschlossene Einheitsvolumen in ml, i die Anzahl der Rollen und n die Rotordrehzahl in U/min ist. Das eingeschlossene Einheitsvolumen ist proportional zum Quadrat des Innendurchmessers d der in die Pumpe eingelegten Schlauchleitung.

Die Figuren 4A und 4B zeigen die Flussraten $Q_A$ bzw. $Q_B$ [l/min] der Pumpe A (Substituatpumpe 21) und der Pumpe B (Blutpumpe 6) als Funktion der Drehzahl $n_A$ bzw. $n_B$ [l/s]. Es zeigt sich, dass unterschiedliche Innendurchmesser $d_A, d_B$ der Schlauchleitungen zu unterschiedlichen Verhältnissen der Drehzahlen $n_A$, $n_B$ der Pumpen A und B führen. Weicht das Verhältnis der Drehzahlen $n_A / n_B$ der Pumpen A und B bei konstantem Systemdruck P voneinander ab, so liegen unterschiedliche Schlauchdurchmesser vor.

Bei Kenntnis des Innendurchmessers $d_A$ bzw. $d_B$ des einen Schlauchleitungsabschnitts kann auf den Innendurchmesser $d_B$ bzw. $d_A$ des anderen Schlauchleitungsabschnitts geschlossen werden, wenn das Verhältnis der Drehzahlen $n_B / n_A$ berechnet wird.

$$d_A = d_B \cdot \sqrt{\frac{n_B}{n_A}}$$

Für die Erkennung des Schlauchsystems kommuniziert die Auswerteinheit 25 mit der zentralen Steuereinheit 24 und empfängt das Drucksignal von der Druckmesseinheit 27, 30. Zunächst iniziiert die zentrale Steuereinheit 24 einen Konnektionstest, um sicherzustellen, dass für den Spülvorgang die Substitutionsflüssigkeitsleitung 18 an der arteriellen Blutleitung 6 angeschlossen ist. Dafür wird die Blutpumpe 10 angehalten, während die Substituatpumpe 21 läuft. Wenn ein positiver Systemdruck P mit der Druckmesseinheit 27, 30 in der Blutleitung 6 gemessen wird, schließt die Auswerteinheit darauf, dass beide Schlauchleitungsabschnitte miteinander verbunden sind. Anschließend steuert die Steuereinheit 24 die Blutpumpe 10 mit einer konstanten Drehzahl $n_B$ an. Daraus ergibt sich eine bestimmte Flussrate $Q_B$, mit der die Blutpumpe 10 Flüssigkeit fördert. Gleichzeitig steuert die Steuereinheit die Substituatpumpe 21 mit einer Drehzahl $n_A$ an, die größer als die Drehzahl $n_A$ der Blutpumpe 10 ist. Dabei wird der Systemdruck P mit der Druckmesseinheit überwacht. Wenn beide Schlauchleitungsabschnitte 18, 6 den gleichen Innendurchmesser hätten, ($d_A = d_B$) kann ein konstanter Systemdruck P nur dann erreicht werden, wenn die Drehzahlen beider Pumpen gleich sind ($n_A = n_B$). Denn nur dann ergäben sich bei gleicher Pumpendrehzahl die gleichen Flussraten $Q_A = Q_B$.

Da beim vorliegenden Ausführungsbeispiel aber der Innendurchmesser $d_B$ der Schlauchleitung des Schlauchleitungsabschnitts 6 größer als der Innendurchmesser $d_A$ der Schlauchleitung des Schlauchleitungsabschnitts 18 ist, muss die Substituatpumpe 21 mit einer höheren Drehzahl $n_A$ betrieben werden, um einen konstanten Systemdruck P einzustellen.

Die zentrale Steuereinheit 24 regelt die Drehzahl der Substituatpumpe 21 solange, bis sich in einem vorgegebenen Zeitintervall (Messphase) ein konstanter Systemdruck P einstellt. Auf Grund der unterschiedlichen Drehzahlen beider Pumpen, die sich bei der Einstellung des konstanten Systemdrucks ergeben, schließt die Auswerteinheit 25 darauf, dass der Schlauchleitungsabschnitt 18 einen kleineren Innendurchmesser $d_A$ als der Schlauchleitungsabschnitt 6 hat. Damit ist das Schlauchleitungssystem erkannt.

Ein konstanter Systemdruck wird in der Praxis auch dann angenommen, wenn das Drucksignal von der Druckmesseinheit 27, 30 von Druckpulsen überlagert ist, die auf die Bewegungen der die Schlauchleitung okkludierenden Rollen der Blutpumpe 10 zurückzuführen sind. Die das Drucksignal auswertende Auswerteinheit 25 berechnet daher bei dem vorliegenden Ausführungsbeispiel den Mittelwert des Drucksignals der Druckmesseinheit 27, 30, wobei die Steuereinheit 24 die Blutpumpe 10 und die Substituatpumpe 21 derart ansteuert, dass sich in einem vorgegebenen Zeitintervall ein konstanter mittlerer Druck einstellt. Das gemessene Drucksignal kann aber auch mit anderen statistischen und/oder signalverarbeitenden Verfahren ausgewertet werden.

Wenn das Schlauchleitungssystem erkannt ist, erzeugt die zentrale Steuereinheit 24 ein Steuersignal, um beispielsweise einen Eingriff in die Maschinensteuerung vorzunehmen. Dieser kann beispielsweise darin liegen, dass mit dem eingelegten Schlauchleitungssystem nur eine Hämodialyse, nicht aber eine Hämofiltrationsbehandlung (Hämodialysefiltrationsbehandlung) möglich ist. Es ist aber auch möglich, die Erkennung des Schlauchleitungssystem dem behandelnden Arzt durch einen optischen und/oder akustischen Hinweis zu signalisieren.

Es sei angenommen, dass ein Schlauchleitungssystem eingelegt ist, deren Schlauchleitungsabschnitte den gleichen Innendurchmesser haben. Dann führt die Nachregelung der Drehzahl $n_A$ der Substituatpumpe 21 durch die Steuereinheit 24 dazu, dass sich für Substituatpumpe 21 und Blutpumpe 10 die gleichen Drehzahlen $n_A = n_B$ ergeben. Dann schließt die Auswerteinheit 25 darauf, dass ein Schlauchleitungssystem mit Schlauchleitungsabschnitten unterschiedlichen Innendurchmessers nicht eingelegt ist. Beispielsweise kann dann die Steuereinheit 24 die Blutbehandlungsvorrichtung für eine Hämofiltration (Hämodiafiltration) freigeben.

Der Vergleich der Drehzahlen $n_A$, $n_B$ der Substituat- und Blutpumpe 21, 10 erfolgt in der Auswerteinheit 25 dadurch, dass die Differenz den Drehzahlen $n_A$ - $n_B$ berechnet wird. Der Betrag der Differenz wird dann mit einem gegebenen Referenzwert verglichen. Wenn der Betrag der Differenz größer als der vorgegebene Referenzwert ist, schließt die Auswerteinheit darauf, dass das Schlauchleitungssystem mit Schlauchleitungsabschnitten unterschiedlichen Innendurchmessers eingelegt ist. Andernfalls wird darauf geschlossen, dass das Schlauchleitungssystem Schlauchleitungsabschnitte mit dem gleichen Innendurchmesser aufweist. Anstelle der Differenz zwischen den beiden Drehzahlen kann auch der Quotient der Drehzahlen gebildet und mit einem vorgegebenen Referenzwert verglichen werden. Allein entscheidend ist, dass die beiden Drehzahlen in ein Verhältnis zueinander gesetzt werden.

Nachfolgend wird eine alternative Ausführungsform der Einrichtung zur Erkennung eines Schlauchleitungssystems beschrieben, mit der zwischen einem Schlauchleitungssystem zur Behandlung eines Erwachsenen oder eines Kindes unterschieden werden kann. Diese alternative Ausführungsform kann zusammen mit der zuvor beschriebenen Ausführungsform oder anstelle der zuvor beschriebenen Ausführungsform in die Blutbehandlungsvorrichtung implementiert sein.

Die Auswerteinheit 25 ist über eine Leitung 13' mit der Bilanziereinheit 13 verbunden, so dass die Auswerteinheit die Bilanztakte der Bilanziereinheit auswerten kann. Die Anzahl der Takte ist ein Maß für die Flussrate der durch die Dialysierflüssigkeitszuführleitung 11 strömenden Flüssigkeit. Da das Volumen der Bilanzkammern bekannt ist, kann die Auswerteinheit aus den Takten der Bilanziereinheit 13 die Flussrate berechnen.

Bei dem vorliegenden Ausführungsbeispiel ist das Schlauchleitungssystem zur Behandlung eines Kindes bestimmt. Sämtliche Schlauchleitungsabschnitte 6, 7, 18 haben den gleichen Innendurchmesser d, der kleiner als der Innendurchmesser der Schlauchleitungen eines Schlauchleitungssystems für die Behandlung eines Erwachsenen ist. Die Auswerteinheit 25 überprüft insbesondere während der Spülphase, ob das richtige Schlauchleitungssystem eingelegt ist. Hierzu steuert die zentrale Steuereinheit 24 die Substituatpumpe 21 mit einer vorgegebenen Drehzahl $n_A$ an. Die Drehzahl $n_A$, mit der die Substituatpumpe betrieben wird, ist diejenige Drehzahl, die für die Einstellung einer bestimmten Flussrate erforderlich ist, wenn das Schlauchsystem für Kinder in die Blutbehandlungsvorrichtung eingelegt ist. Während die Substituatpumpe 21 läuft, überwacht die Auswerteinheit 25 die tatsächliche Flussrate Q, mit der die Substituatpumpe 21 Spülflüssigkeit ansaugt. Wenn die mit der Bilanziereinheit 13 gemessene Flussrate von der angenommenen Flussrate um einen Betrag abweicht, der größer als ein Referenzwert ist, schließt die Auswerteinheit 25 darauf, dass in die Blutbehandlungsvorrichtung nicht ein Schlauchsystem für Kinder eingelegt ist. Im vorliegenden Fall ist aber das richtige Schlauchleitungssystem für Kinder eingelegt. Daher ist die gemessene Flussrate im Wesentlichen gleich der angenommenen Flussrate, d.h. die Differenz beider Flussraten ist kleiner als ein vorgegebener Referenzwert, so dass die Auswerteinheit darauf schließt, dass ein Schlauchsystem für Kinder eingelegt ist. Die Steuereinheit 25 erzeugt daraufhin wieder ein Steuersignal, so dass ein Eingriff in die Maschinensteuerung vorgenommen werden kann. Beispielsweise können bestimmte Vorgaben für die Blutbehandlung gemacht werden, die für die Behandlung eines Kindes erforderlich sind. Es ist aber auch möglich, die Erkennung des Schlauchleitungssystems dem behandelnden Arzt durch einen optischen und/oder akustischen Hinweis zu signalisieren.

**Patentansprüche**

1. Vorrichtung zur extrakorporalen Blutbehandlung mit einem Dialysator (1) oder Filter, der eine erste und eine zweite Kammer (3, 4) aufweist, die von einer semipermeablen Membran getrennt sind, mindestens einer mit einer vorgegebenen Drehzahl n zu betreibenden Pumpe (21, 10) zum Fördern von Flüssigkeit in einem in die extrakorporale Blutbehandlungsvorrichtung einzulegenden Schlauchleitungssystems (5, 6, 18), und einer zentralen Steuereinheit (24) zum Steuern der extrakorporalen Blutbehandlungsvorrichtung, wobei die extrakorporale Blutbehandlungsvorrichtung eine Einrichtung (25, 26, 27) zur Erkennung des in die extrakorporale Blutbehandlungsvorrichtung einzulegenden Schlauchleitungssystems aufweist, **dadurch gekennzeichnet,** **dass** die Einrichtung (25, 26, 27) zur Erkennung des Schlauchleitungssystems aufweist:

   eine Auswerteinheit (25), die derart ausgebildet ist, dass das Schlauchleitungssystem auf der Grundlage der Abhängigkeit der Flussrate Q, mit der die Flüssigkeit von der mindestens einen Pumpe (21, 10) in dem Schlauchleitungsabschnitt (18, 18'; 6, 6') gefördert wird, von der Drehzahl n, mit der die mindestens eine Pumpe betrieben wird, und dem Innendurchmesser d des Schlauchleitungsabschnitts (18, 18'; 6, 6') erkannt wird.

2. Vorrichtung nach Anspruch 1, **dadurch gekennzeichnet, dass** die Auswerteinheit (25) mit der zentralen Steuereinheit (24) zusammenwirkt, wobei die Auswerteinheit (25) nach der Erkennung des Schlauchleitungssystems ein Steuersignal für die zentrale Steuereinheit erzeugt, so dass die Steuereinheit einen Eingriff in die Maschinensteuerung vornimmt.

3. Vorrichtung nach Anspruch 1 oder 2, **dadurch gekennzeichnet, dass** die mindestens eine Pumpe (21, 10) eine peristaltische Pumpe ist, in die ein Schlauchleitungsabschnitt (18, 6) des Schlauchleitungssystems (6, 8, 18) eingelegt ist.

4. Vorrichtung nach einem der Ansprüche 1 bis 3, **dadurch gekennzeichnet, dass** eine erste Pumpe (21) zum Fördern von Flüssigkeit in einem ersten Schlauchleitungsabschnitt (18) und eine zweite Pumpe (10) zum Fördern von Flüssigkeit in einem zweiten Schlauchleitungsabschnitt (6) des Schlauchsystems (6, 8, 18) vorgesehen sind, wobei der erste und zweite Schlauchleitungsabschnitt (18, 6) hintereinander angeordnet sind und der erste Schlauchleitungsabschnitt (18) einen ersten Innendurchmesser $d_A$ und der zweite Schlauchleitungsabschnitt (6) einen zweiten Innendurchmesser $d_B$ aufweist, der gleich dem ersten Innendurchmesser ist oder von dem ersten Innendurchmesser verschieden ist, und dass die Einrichtung zur Erkennung des Schlauchleitungssystems eine Einheit (26, 27) zum Messen des Drucks in der Schlauchleitung zwischen der ersten und der zweiten Pumpe (21, 10) aufweist, wobei die Auswerteinheit (25) mit der Steuereinheit (24) und der Druckmesseinheit (27, 30) derart zusammenwirkt, dass mit der Auswerteinheit das Verhältnis der Drehzahlen der ersten und zweiten Pumpe ermittelt wird, bei dem sich der mit der Druckmesseinheit gemessene Druck in einem vorgegebenen Zeitintervall nicht verändert, und dass für den Fall, dass die Drehzahl der ersten und zweiten Pumpe nicht gleich sind, darauf geschlossen wird, dass der Innendurchmesser des ersten und zweiten Schlauchleitungsabschnitts voneinander verschieden sind, und dass für den Fall, dass die Drehzahl der ersten und zweiten Pumpe im Wesentlichen gleich sind, darauf geschlossen wird, dass der Innendurchmesser des ersten und zweiten Schlauchleitungsabschnitts gleich ist.

5. Vorrichtung nach Anspruch 4, **dadurch gekennzeichnet, dass** die Auswerteinheit (25) mit der Steuereinheit (24) und der Druckmesseinheit (27, 30) derart zusammenwirkt, dass in einem ersten Schritt die zweite Pumpe mit einer vorgegebenen Drehzahl betrieben wird, so dass in dem zweiten Schlauchleitungsabschnitt Flüssigkeit mit einer vorgegebenen Flussrate gefördert wird, und die erste Pumpe mit einer vorgegebenen ersten Drehzahl betrieben wird, die größer als die Drehzahl der zweiten Pumpe ist, und dass in einem zweiten Schritt der Druck in der Schlauchleitung zwischen der ersten und zweiten Pumpe überwacht und die Drehzahl der ersten Pumpe solange bis zu einer zweiten Drehzahl verändert wird, bei der der Druck in der Schlauchleitung zwischen der ersten und zweiten Pumpe konstant bleibt, wobei von der Auswerteinheit darauf geschlossen wird, dass der Innendurchmesser der ersten Schlauchleitung kleiner als der Innendurchmesser der zweiten Schlauchleitung ist, wenn die zweite Drehzahl der ersten Pumpe größer als die Drehzahl der zweiten Pumpe ist, und darauf geschossen wird, dass der Innendurchmesser der ersten Schlauchleitung gleich dem Innendurchmesser der zweiten Schlauchleitung ist, wenn die zweite Drehzahl der ersten Pumpe im Wesentlichen gleich der Drehzahl der zweiten Pumpe ist.

6. Vorrichtung nach einem der Ansprüche 1 bis 3, **dadurch gekennzeichnet, dass** die Einrichtung zur Erkennung des Schlauchleitungssystems eine Einheit (13) zum Messen der Flussrate der mindestens einen Pumpe (21) in dem Schlauchleitungsabschnitt (18) der Schlauchleitung aufweist, und dass die Auswerteinheit (25) mit der Steuereinheit (24) und der Einheit (13) zum Messen der Flussrate derart zusammenwirkt, dass die mindestens eine Pumpe (21) mit einer vorgegebenen Drehzahl betrieben wird, bei der in einer bestimmten Schlauchleitung, die einen vorgegebenen Innendurchmesser aufweist, die Flüssigkeit mit einer bestimmten Flussrate gefördert wird, und dass die vorgegebene Flussrate mit der Flussrate verglichen wird, die von der Einheit (13) zum Messen der Flussrate gemessen wird, wobei von der Auswerteinheit (25) darauf geschlossen wird, dass der Innendurchmesser der Schlauchleitung von dem Innendurchmesser der bestimmten Schlauchleitung verschieden ist, wenn die mit der eingestellten Drehzahl vorgegebene Flussrate von der gemessenen Flussrate um einen Betrag abweicht, der größer als ein Referenzwert ist, und darauf geschossen wird, dass der Innendurchmesser der Schlauchleitung gleich dem Innendurchmesser der bestimmten Schlauchleitung ist, wenn die mit der eingestellten Drehzahl vorgegebene Flussrate von der gemessenen Flussrate nicht um einen Betrag abweicht, der größer als der Referenzwert ist.

7. Verfahren zum Erkennen eines in eine extrakorporale Blutbehandlungsvorrichtung einzulegenden Schlauchleitungssystems, wobei die extrakorporale Blutbehandlungsvorrichtung aufweist:

   einen Dialysator oder Filter, der eine erste und eine zweite Kammer aufweist, die von einer semipermeablen Membran getrennt sind, mindestens eine mit einer vorgegebenen Drehzahl zu betreibenden Pumpe zum Fördern von Flüssigkeit in einem in die extrakorporale Blutbehandlungsvorrichtung einzulegenden Schlauchleitungssystems, und

eine zentralen Steuereinheit zum Steuern der extrakorporalen Blutbehandlungsvorrichtung, **dadurch gekennzeichnet,** **dass** das Schlauchleitungssystem auf der Grundlage der Abhängigkeit der Flussrate, mit der die Flüssigkeit von der mindestens einen Pumpe in dem Schlauchleitungsabschnitt gefördert wird, von der Drehzahl, mit der die mindestens eine Pumpe betrieben wird, und dem Innendurchmesser des Schlauchleitungsabschnitts erkannt wird.

8.  Verfahren nach Anspruch 7, **dadurch gekennzeichnet, dass** nach der Erkennung des Schlauchleitungssystems ein Steuersignal für die zentrale Steuereinheit erzeugt, so dass die Steuereinheit einen Eingriff in die Maschinensteuerung vornimmt.

9.  Verfahren nach Anspruch 7 oder 8, **dadurch gekennzeichnet, dass** die mindestens eine Pumpe eine peristaltische Pumpe ist, in die ein Schlauchleitungsabschnitt des Schlauchleitungssystems eingelegt wird.

10. Verfahren nach einem der Ansprüche 7 bis 9, **dadurch gekennzeichnet, dass** eine erste Pumpe zum Fördern von Flüssigkeit in einem ersten Schlauchleitungsabschnitt und eine zweite Pumpe zum Fördern von Flüssigkeit in einem zweiten Schlauchleitungsabschnitt des Schlauchsystems vorgesehen sind, wobei der erste und zweite Schlauchleitungsabschnitt hintereinander angeordnet sind und der erste Schlauchleitungsabschnitt einen ersten Innendurchmesser und der zweite Schlauchleitungsabschnitt einen zweiten Innendurchmesser aufweist, der gleich dem ersten Innendurchmesser ist oder von dem ersten Innendurchmesser verschieden ist, und
dass der Druck in der Schlauchleitung zwischen der ersten und der zweiten Pumpe gemessen und das Verhältnis der Drehzahlen der ersten und zweiten Pumpe ermittelt wird, bei dem sich der gemessene Druck in einem vorgegebenen Zeitintervall nicht verändert, und dass für den Fall, dass die Drehzahl der ersten und zweiten Pumpe nicht gleich sind, darauf geschlossen wird, dass der Innendurchmesser des ersten und zweiten Schlauchleitungsabschnitts voneinander verschieden sind, und dass für den Fall, dass die Drehzahl der ersten und zweiten Pumpe im Wesentlichen gleich sind, darauf geschlossen wird, dass der Innendurchmesser des ersten und zweiten Schlauchleitungsabschnitts gleich ist.

11. Verfahren nach Anspruch 10, **dadurch gekennzeichnet, dass** in einem ersten Schritt die zweite Pumpe mit einer vorgegebenen Drehzahl betrieben wird, so dass in dem zweiten Schlauchleitungsabschnitt Flüssigkeit mit einer vorgegebenen Flussrate gefördert wird, und die erste Pumpe mit einer vorgegebenen ersten Drehzahl betrieben wird, die größer als die Drehzahl der zweiten Pumpe ist, und dass in einem zweiten Schritt der Druck in der Schlauchleitung zwischen der ersten und zweiten Pumpe überwacht und die Drehzahl der ersten Pumpe solange bis zu einer zweiten Drehzahl verändert wird, bei der der Druck in der Schlauchleitung zwischen der ersten und zweiten Pumpe konstant bleibt, wobei darauf geschlossen wird, dass der Innendurchmesser der ersten Schlauchleitung kleiner als der Innendurchmesser der zweiten Schlauchleitung ist, wenn die zweite Drehzahl der ersten Pumpe größer als die Drehzahl der zweiten Pumpe ist, und darauf geschossen wird, dass der Innendurchmesser der ersten Schlauchleitung gleich dem Innendurchmesser der zweiten Schlauchleitung ist, wenn die zweite Drehzahl der ersten Pumpe im Wesentlichen gleich der Drehzahl der zweiten Pumpe ist.

12. Verfahren nach Anspruch 7, **dadurch gekennzeichnet, dass** die Flussrate der mindestens einen Pumpe in dem Schlauchleitungsabschnitt der Schlauchleitung gemessen und die mindestens eine Pumpe mit einer vorgegebenen Drehzahl betrieben wird, bei der in einer bestimmten Schlauchleitung, die einen vorgegebenen Innendurchmesser aufweist, die Flüssigkeit mit einer bestimmten Flussrate gefördert wird, und dass die vorgegebene Flussrate mit der gemessenen Flussrate verglichen wird, wobei darauf geschlossen wird, dass der Innendurchmesser der Schlauchleitung von dem Innendurchmesser der bestimmten Schlauchleitung verschieden ist, wenn die mit der eingestellten Drehzahl vorgegebene Flussrate von der gemessenen Flussrate um einen Betrag abweicht, der größer als ein Referenzwert ist, und darauf geschossen wird, dass der Innendurchmesser der Schlauchleitung gleich dem Innendurchmesser der bestimmten Schlauchleitung ist, wenn die mit der eingestellten Drehzahl vorgegebene Flussrate von der gemessenen Flussrate nicht um einen Betrag abweicht, der größer als der Referenzwert ist.

**Claims**

1.  Apparatus for extracorporeal blood treatment, having
a dialyzer (1) or filter that has a first and a second chamber (3, 4), which chambers are separated by a semi-permeable membrane,
at least one pump (21, 10) to be operated at a predetermined speed of rotation n, for conveying fluid in a tubing system (5, 6, 18) to be laid into the extracorporeal blood treatment apparatus, and
a central control unit (24) for controlling the extracorporeal blood treatment apparatus,

wherein the extracorporeal blood treatment apparatus has a device (25, 26, 27) for identifying the tubing system to be laid into the extracorporeal blood treatment apparatus, **characterized in that** the device (25, 26, 27) has the following for identifying the tubing system:

an evaluation unit (25) that is configured in such a manner that the tubing system is identified on the basis of the dependence of the flow rate Q at which the fluid is conveyed in the tubing section (18, 18'; 6, 6') by the at least one pump (21, 10) on the speed of rotation n at which the at least one pump is operated, and the inside diameter d of the tubing section (18, 18'; 6, 6').

2. Apparatus according to claim 1, **characterized in that** the evaluation unit (25) works together with the central control unit (24), wherein the evaluation unit (25) generates a control signal for the central control unit after identifying the tubing system, so that the control unit undertakes an intervention in the machine control.

3. Apparatus according to claim 1 or 2, **characterized in that** the at least one pump (21, 10) is a peristaltic pump into which a tubing section (18, 6) of the tubing system (6, 8, 18) is laid.

4. Apparatus according to one of claims 1 to 3, **characterized in that** a first pump (21) for conveying fluid in a first tubing section (18) and a second pump (10) for conveying fluid in a second tubing section (6) of the tubing system (6, 8, 18) are provided, wherein the first and second tubing section (18, 6) are disposed one behind the other, and the first tubing section (18) has a first inside diameter $d_A$ and the second tubing section (6) has a second inside diameter $d_B$ that is equal to the first inside diameter or different from the first inside diameter, and that the device for identifying the tubing system has a unit (26, 27) for measuring the pressure in the tubing between the first and the second pump (21, 10), wherein the evaluation unit (25) works together with the control unit (24) and the pressure measuring unit (27, 30) in such a manner that the ratio of the speeds of rotation of the first and second pump is determined using the evaluation unit, at which ratio the pressure measured using the pressure measuring unit does not change over a predetermined time interval, and that in the event that the speeds of rotation of the first and second pump are not the same, the conclusion is drawn that the inside diameters of the first and second tubing section are different from one another, and that in the event that the speeds of rotation of the first and second pump are essentially the same, the conclusion is drawn that the inside diameters of the first and second tubing section are the same.

5. Apparatus according to claim 4, **characterized in that** the evaluation unit (25) works together with the control unit (24) and the pressure measuring unit (27, 30) in such a manner that in a first step, the second pump is operated at a predetermined speed of rotation, so that fluid is conveyed in the second tubing section at a predetermined flow rate, and the first pump is operated at a predetermined first speed of rotation that is greater than the speed of rotation of the second pump, and that in a second step, the pressure in the tubing between the first and second pump is monitored, and the speed of rotation of the first pump is changed continuously, up to a second speed of rotation at which the pressure in the tubing between the first and second pump remains constant, whereupon the conclusion is drawn by the evaluation unit that the inside diameter of the first tubing is smaller than the inside diameter of the second tubing if the second speed of rotation of the first pump is greater than the speed of rotation of the second pump, and the conclusion is drawn that the inside diameter of the first tubing is equal to the inside diameter of the second tubing if the second speed of rotation of the first pump is essentially equal to the speed of rotation of the first pump.

6. Apparatus according to one of claims 1 to 3, **characterized in that** the device for identifying the tubing system has a unit (13) for measuring the flow rate of the at least one pump (21) in the tubing section (18) of the tubing, and that the evaluation unit (25) works together with the control unit (24) and the unit (13) for measuring the flow rate, in such a manner that the at least one pump (21) is operated at a predetermined speed of rotation at which the fluid is conveyed at a specific flow rate in a specific tubing that has a predetermined inside diameter, and that the predetermined flow rate is compared with the flow rate that is measured by the unit (13) for measuring the flow rate, whereupon the conclusion is drawn by the evaluation unit that the inside diameter of the tubing is different from the inside diameter of the specific tubing if the flow rate predetermined with the set speed of rotation deviates from the measured flow rate by an amount that is greater than a reference value, and the conclusion is drawn that the inside diameter of the tubing is equal to the inside diameter of the specific tubing if the flow rate predetermined with the set speed of rotation does not differ from the measured flow rate by an amount that is greater than the reference value.

7. Method for identifying a tubing system to be laid into an extracorporeal blood treatment apparatus,

wherein the extracorporeal blood treatment apparatus comprises:

a dialyzer or filter that has a first and a second chamber, which chambers are separated by a semi-permeable membrane,
at least one pump to be operated at a predetermined speed of rotation, for conveying fluid in a tubing system to be laid into the extracorporeal blood treatment apparatus, and
a central control unit for controlling the extracorporeal blood treatment apparatus,
**characterized in that**
the tubing system is identified on the basis of the dependence of the flow rate at which the fluid is conveyed in the tubing section by the at least one pump on the speed of rotation at which the at least one pump is operated, and the inside diameter of the tubing section.

8. Method according to claim 7, **characterized in that** after identification of the tubing system, a control signal for the central control unit [word missing: is] generated, so that the control unit undertakes an intervention in the machine control.

9. Method according to claim 7 or 8, **characterized in that** the at least one pump is a peristaltic pump into which a tubing section of the tubing system is laid.

10. Method according to one of claims 7 to 9, **characterized in that** a first pump is provided for conveying fluid in a first tubing section and a second pump is provided for conveying fluid in a second tubing section of the tubing system, wherein the first and second tubing section are disposed one behind the other, and the first tubing section has a first inside diameter and the second tubing section has a second inside diameter that is equal to the first inside diameter or different from the first inside diameter, and that the pressure in the tubing between the first and the second pump is measured and the ratio of the speeds of rotation of the first and second pump is determined, at which ratio the measured pressure does not change during a predetermined time interval, and that in the event that the speeds of rotation of the first and second pump are not the same, the conclusion is drawn that the inside diameters of the first and second tubing section are different from one another, and that in the event that the speeds of rotation of the first and second pump are essentially the same, the conclusion is drawn that the inside diameters of the first and second tubing section are the same.

11. Method according to claim 10, **characterized in that** in a first step, the second pump is operated at a predetermined speed of rotation, so that fluid is conveyed in the second tubing section at a predetermined flow rate, and the first pump is operated at a predetermined first speed of rotation that is greater than the speed of rotation of the second pump, and that in a second step, the pressure in the tubing between the first and second pump is monitored, and the speed of rotation of the first pump is changed continuously, up to a second speed of rotation at which the pressure in the tubing between the first and second pump remains constant, whereupon the conclusion is drawn that the inside diameter of the first tubing is smaller than the inside diameter of the second tubing if the second speed of rotation of the first pump is greater than the speed of rotation of the second pump, and the conclusion is drawn that the inside diameter of the first tubing is equal to the inside diameter of the second tubing if the second speed of rotation of the first pump is essentially equal to the speed of rotation of the first pump.

12. Method according to claim 7, **characterized in that** the flow rate of the at least one pump is measured in the tubing section of the tubing and the at least one pump is operated at a predetermined speed of rotation at which the fluid is conveyed at a specific flow rate in a specific tubing that has a predetermined inside diameter, and that the predetermined flow rate is compared with the measured flow rate, whereupon the conclusion is drawn that the inside diameter of the tubing is different from the inside diameter of the specific tubing if the flow rate predetermined with the set speed of rotation deviates from the measured flow rate by an amount that is greater than a reference value, and the conclusion is drawn that the inside diameter of the tubing is equal to the inside diameter of the specific tubing if the flow rate predetermined with the set speed of rotation does not differ from the measured flow rate by an amount that is greater than the reference value.

## Revendications

1. Dispositif destiné au traitement extracorporel du sang comprenant :

un dialyseur (1) ou filtre qui présente un premier et un deuxième compartiments (3, 4) qui sont séparés par une membrane semi-perméable,
au moins une pompe (21, 10) devant être exploitée à un régime prédéfini n destinée à faire circuler un liquide dans un système de conduites souples (5, 6, 18) devant être intégré dans le dispositif de traitement extracorporel du sang, et
une unité de commande centrale (24) destinée à commander le dispositif de traitement extracorporel du sang,
dans lequel le dispositif de traitement extracorporel du sang comprend un dispositif (25, 26, 27) destiné à reconnaître le système de conduites souples devant être intégré dans le dispositif de traitement extracorporel du sang,

**caractérisé**

**en ce que** le dispositif (25, 26, 27) destiné à reconnaître le système de conduites souples présente :

une unité d'analyse (25) qui est conçue de telle sorte que le système de conduites souples est reconnu sur la base de la dépendance du débit Q auquel le liquide est déplacé dans une section de conduite souple (18, 18' ; 6, 6') par l'au moins une pompe (21, 10) par rapport au régime n auquel l'au moins une pompe est exploitée et au diamètre intérieur d de la section de conduite souple (18, 18' ; 6, 6').

2. Dispositif selon la revendication 1, **caractérisé en ce que** l'unité d'analyse (25) coopère avec l'unité de commande centrale (24), l'unité d'analyse (25), après la reconnaissance du système de conduites souples, produisant un signal de commande pour l'unité de commande centrale, de telle sorte que l'unité de commande effectue une intervention dans la commande de la machine.

3. Dispositif selon la revendication 1 ou 2, **caractérisé en ce que** l'au moins une pompe (21, 10) est une pompe péristaltique dans laquelle une section de conduite souple (18, 6) du système de conduites souples (6, 8, 18) est insérée.

4. Dispositif selon l'une des revendications 1 à 3, **caractérisé en ce qu'**il est prévu une première pompe (21) destinée à faire circuler un liquide dans une première section de conduite souple (18) et une deuxième pompe (10) destinée à faire circuler un liquide dans une deuxième section de conduite souple (6) du système de conduites (6, 8, 18), les première et deuxième sections de conduites souples (18, 6) étant agencées l'une derrière l'autre et la première section de conduite souple (18) présentant un premier diamètre intérieur $d_A$ et la deuxième section de conduite souple (6) présentant un deuxième diamètre intérieur $d_B$ qui est identique au premier diamètre intérieur ou différent du premier diamètre intérieur, et **en ce que** le dispositif destiné à reconnaître le système de conduites souples comprend une unité (26, 27) destinée à mesurer la pression dans la conduite souple entre la première et la deuxième pompes (21, 10),

dans lequel l'unité d'analyse (25) coopère avec l'unité de commande (24) et l'unité de mesure de la pression (27, 30) de telle sorte qu'avec l'unité d'analyse, on détermine le rapport entre les régimes des première et deuxième pompes auquel la pression mesurée avec l'unité de mesure de la pression ne varie pas au cours d'un intervalle de temps prédéfini, et que dans le cas où les régimes des première et deuxième pompes ne sont pas identiques, il est conclu que les diamètres intérieurs des première et deuxième sections de conduites souples sont différents l'un de l'autre, et que dans le cas où les régimes des première et deuxième pompes sont essentiellement identiques, il est conclu que les diamètres intérieurs des première et deuxième sections de conduites souples sont identiques.

5. Dispositif selon la revendication 4, **caractérisé en ce que** l'unité d'analyse (25) coopère avec l'unité de commande (24) et l'unité de mesure de la pression (27, 30) de telle sorte que :

dans une première étape, la deuxième pompe est exploitée à un régime prédéfini de telle sorte que le liquide circule dans la deuxième section de conduite souple à un débit prédéfini et la première pompe est exploitée à un premier régime prédéfini qui est supérieur au régime de la deuxième pompe, et

dans une deuxième étape, la pression dans la conduite souple entre les première et deuxième pompes est observée et le régime de la première pompe est modifié jusqu'à un deuxième régime auquel la pression dans la conduite souple entre les première et deuxième pompes reste constante, étant entendu qu'au niveau de l'unité d'analyse, il est conclu que le diamètre intérieur de la première conduite souple est plus petit que le diamètre intérieur de la deuxième conduite souple si le deuxième régime de la première pompe est supérieur au régime de la deuxième pompe et il est conclu que le diamètre intérieur de la première conduite souple est identique au diamètre intérieur de la deuxième conduite souple si le deuxième régime de la première pompe est essentiellement identique au régime de la deuxième pompe.

6. Dispositif selon l'une des revendications 1 à 3, **caractérisé en ce que** le dispositif destiné à reconnaître le système de conduites souples présente une unité (13) destinée à mesurer le débit de l'au moins une pompe (21) dans la section de conduite souple (18) de la conduite souple, et **en ce que** l'unité d'analyse (25) coopère avec l'unité de commande (24) et l'unité (13) de mesure du débit de telle sorte que l'au moins une pompe (21) est exploitée à un régime prédéfini auquel, dans une conduite souple déterminée qui présente un diamètre intérieur prédéfini, le liquide circule à un débit déterminé et que le débit prédéfini est comparé avec le débit qui est mesuré par l'unité (13) de mesure du débit, étant entendu qu'au niveau de l'unité d'analyse (25), il est conclu que le diamètre intérieur de la conduite souple est différent du diamètre intérieur de la conduite souple déterminée si le débit prédéfini au régime réglé diffère du débit mesuré d'une valeur qui est supérieure à une valeur de référence, et il est conclu que le diamètre intérieur de la conduite souple est identique au diamètre intérieur de la conduite souple déterminée si le débit prédéfini au régime réglé ne diffère pas du débit mesuré d'une valeur qui est supérieure à une valeur de référence.

7. Procédé destiné à reconnaître un système de conduites souples devant être intégré dans un dispositif de traitement extracorporel du sang, dans lequel le dispositif de traitement extracorporel du sang présente :

un dialyseur ou filtre qui présente un premier et un deuxième compartiments qui sont séparés par une membrane semi-perméable,
au moins une pompe devant être exploitée à un régime prédéfini destinée à faire circuler un liquide dans un système de conduites souples devant être intégré dans le dispositif de traitement extracorporel du sang, et
une unité de commande centrale destinée à commander le dispositif de traitement extracorporel du sang,
**caractérisé**
**en ce que** le système de conduites souples est reconnu sur la base de la dépendance du débit auquel le liquide est déplacé dans la section de conduite souple par l'au moins une pompe par rapport au régime auquel l'au moins une pompe est exploitée et au diamètre intérieur de la section de conduite souple.

8. Procédé selon la revendication 7, **caractérisé en ce qu'**après la reconnaissance du système de conduites souples, un signal de commande est produit pour l'unité de commande centrale, de telle sorte que l'unité de commande effectue une intervention dans la commande de la machine.

9. Procédé selon la revendication 7 ou 8, caractérisé en ce l'au moins une pompe est une pompe péristaltique dans laquelle une section de conduite souple du système de conduites souples est insérée.

10. Procédé selon l'une des revendications 7 à 9, **caractérisé en ce qu'**il est prévu une première pompe destinée à faire circuler un liquide dans une première section de conduite souple et une deuxième pompe destinée à faire circuler un liquide dans une deuxième section de conduite souple du système de conduites, les première et deuxième sections de conduites souples étant agencées l'une derrière l'autre et la première section de conduite souple présentant un premier diamètre intérieur et la deuxième section de conduite souple présentant un deuxième diamètre intérieur qui est identique au premier diamètre intérieur ou différent du premier diamètre intérieur, et **en ce qu'**on mesure la pression dans la conduite souple entre les première et deuxième pompes et on détermine le rapport entre les régimes des première et deuxième pompes auquel la pression mesurée ne varie pas au cours d'un intervalle de temps prédéfini, et **en ce que** dans le cas où les régimes des première et deuxième pompes ne sont pas identiques, il est conclu que les diamètres intérieurs des première et deuxième sections de conduites souples sont différents l'un de l'autre, et **en ce que** dans le cas où les régimes des première et deuxième pompes sont essentiellement identiques, il est conclu que les diamètres intérieurs des première et deuxième sections de conduites souples sont identiques.

11. Procédé selon la revendication 10, **caractérisé en ce que** dans une première étape, la deuxième pompe est exploitée à un régime prédéfini de telle sorte que le liquide circule dans la deuxième section de conduite souple à un débit prédéfini et la première pompe est exploitée à un premier régime prédéfini qui est supérieur au régime de la deuxième pompe, et
**en ce que** dans une deuxième étape, la pression dans la conduite souple entre les première et deuxième pompes est observée et le régime de la première pompe est modifié jusqu'à un deuxième régime auquel la pression dans la conduite souple entre les première et deuxième pompes reste constante, étant entendu qu'il est conclu que le diamètre intérieur de la première conduite souple est plus petit que le diamètre intérieur de la deuxième conduite souple si le deuxième régime de la première pompe est supérieur au régime de la deuxième pompe et il est conclu que le diamètre intérieur de la première conduite souple est identique au diamètre intérieur de la deuxième conduite souple si le deuxième régime de la première pompe est essentiellement identique au régime de la deuxième pompe.

12. Procédé selon la revendication 7, **caractérisé en ce que** le débit de l'au moins une pompe dans la section de conduite souple de la conduite souple est mesuré et l'au moins une pompe est exploitée à un régime prédéfini auquel dans une conduite souple déterminée qui présente un diamètre intérieur prédéfini, le liquide circule à un débit déterminé et **en ce que** le débit prédéfini est comparé avec le débit mesuré, étant entendu qu'il est conclu que le diamètre intérieur de la conduite souple est différent du diamètre intérieur de la conduite souple déterminée si le débit prédéfini au régime réglé diffère du débit mesuré d'un degré qui est supérieur à une valeur de référence, et il est conclu que le diamètre intérieur de la conduite souple est identique au diamètre intérieur de la conduite souple déterminée si le débit prédéfini au régime réglé ne diffère pas du débit mesuré d'un degré qui est supérieur à une valeur de référence.

**Fig. 1**

Fig. 2

$$P = \text{const} \Rightarrow Q_A = Q_B$$

$$Q_A \cong d_A^2 \cdot n_A \qquad \qquad Q_B \cong d_B^2 \cdot n_B$$

21

P

30, 27

10

18

6

**Fig. 3**

$Q_A$[l/min]

3
2
1

1   3   5   7   $n_A$[l/s]

**Fig. 4A**

$Q_B$[l/min]

3
2
1

1   3   5   7   $n_B$[l/s]

**Fig. 4B**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1112099 B1